# EUROPÄISCHE PATENTSCHRIFT

(11) **EP 2 676 625 B1**
(45) Veröffentlichungstag und Bekanntmachung des Hinweises auf die Patenterteilung: **22.04.2020**
(21) Anmeldenummer: 13168586.9
(22) Anmeldetag: 21.05.2013
(51) Int. Cl.: A61B 18/14

(54) **HF-Versiegelungsinstrument**
HF sealing instrument
Instrument de scellage HF

(30) Priorität: 18.06.2012 DE 102012105263
(43) Veröffentlichungstag der Anmeldung: 25.12.2013
(73) Patentinhaber: AESCULAP AG, 78532 Tuttlingen (DE)
(72) Erfinder: Rothweiler, Christoph, 78166 Donaueschingen (DE); Heizmann, Patrick, 78183 Hüfingen (DE)
(74) Vertreter: Winter, Brandl, Fürniss, Hübner, Röss, Kaiser, Polte - Partnerschaft mbB

(56) Entgegenhaltungen:
- EP-A1- 2 436 330
- DE-A1- 2 455 171
- DE-A1-102006 042 985
- DE-A1-102008 032 511

## Beschreibung

Die vorliegende Erfindung betrifft ein chirurgisches, bipolares HF-Instrument, insbesondere ein bipolares HF-Versiegelungsinstrument gemäß dem Anspruch 1.

### Hintergrund der Erfindung

Moderne chirurgische HF-Instrumente der Bipolarbauart ermöglichen heutzutage neue Verfahren sowie chirurgische Arbeitsmethoden, um Gefäße und Gewebestrukturen dauerhaft und sicher zu thermofusionieren. Die hohe Sicherheit dieser Instrumente bei der Versiegelung eröffnen hierbei weite Anwendungsmöglichkeiten auch in der Minimal - Invasivtechnik mit besonders kleinen Zugangswegen, wodurch OP - Zeiten eingespart und OP - Kosten gesenkt werden können.

Die Thermofusion basiert dabei im Wesentlichen auf zwei Mechanismen, die für den Erhalt eines guten Fusionsergebnisses optimal aufeinander abgestimmt sein müssen, nämlich
- zum Einen auf der Stromapplikation über zwei sich gegenüberliegenden Elektroden - bestückte Instrumentenbranchen, zwischen denen das zu behandelnde Gewebe eingespannt wird und
- zum Anderen auf dem Anpressdruck (Einspannkraft), mit welchem das zu behandelnde Gewebe zwischen den Instrumentenbranchen komprimiert wird.

Beide Faktoren beeinflussen und verändern die Kollagen- und Elastinstrukturen physikalisch und erzeugen auch die pergamentartige Thermofusionszone, die den sicheren Verschluss der Gefäße optisch erkennen lässt.

Die grundlegende chirurgische Vorgehensweise bei der Thermofusion mittels eines HF - Versiegelungsinstruments bekannter Bauart lässt sich für alle bekannten Instrumente einheitlich wie folgt umschreiben:
Das zu durchtrennende und zu versiegelnde Gewebe wird zunächst mit den zwei Branchen des Instruments erfasst und dazwischen mit einer definierten Klemmkraft eingespannt. Anschließend wird der Thermofusionsmodus per Hand- oder Fußschalter aktiviert, worauf die Elektroden der einen Branche mit einem HF - Strom beaufschlagt werden, der durch das eingeklemmte Gewebe hindurch zur Elektrode der gegenüberliegenden Branche geleitet wird und dabei das Gewebe thermofusioniert. Die Stromzufuhr wird entweder manuell oder automatisch beendet, sobald die optimale Fusionsqualität erreicht ist. Aufgrund des bipolaren Instrumentenaufbaus werden hierbei laterale thermische Schädigungen weitgehend vermieden. Abschließend erfolgt in der Mitte der Fusionszone ein Durchschneiden des Gewebes mittels eines (mechanischen oder elektrischen) Skalpells oder einer scharfkantigen Zange als weiterer Bestandteil des Instruments.

### Stand der Technik

In der Praxis werden HF - Versiegelungsinstrumente der Bipolarbauart beispielsweise für die vaginale Hysterektomie oder Thyreoidetomie in Kleinschnitttechnik erfolgreich eingesetzt. Aber auch in anderen Disziplinen wie z.B. der Darmanastomose ersetzen bipolare HF-Versiegelungsinstrumente zunehmend bekannte Standardinstrumente wie das Klammernahtinstrument oder auch Stapler genannt. In diesem besonderen Fall erfordert die offenchirurgische OP - Technik zum Anlegen einer Darmanastomose u.a. eine Teilbarkeit des Instruments in zwei unabhängig voneinander frei bewegbare Instrumententeile. Wird demnach ein HF-Versiegelungsinstrument der Bipolarbauart eingesetzt, erfolgt die Verbindung der Gewebeteile zur Anastomosierung nicht durch Klammern, sondern die Gewebeteile werden durch einen hochfrequenten Strom sozusagen "verschweißt". Dennoch soll/muss das Instrument teilbar und die beiden Instrumentenhälften vollständig und unabhängig voneinander bewegbar sein.

Der Strom muss dabei von der einen Instrumentenhälfte mit den in der Branche einliegenden Elektroden über das eingespannte Gewebe zur Gegenelektrode fließen, welche sich in der Branche der anderen Instrumentenhälfte befindet. Um verschiedene Gewebestärken versiegeln zu können, muss darüber hinaus eine Kontaktierung der beiden Instrumentenhälften auch unter verschiedenen Elektrodenabständen etwa zwischen 0.1mm und 1.5mm möglich sein.

Um unter diesen Randbedingungen ein optimales Versiegelungsergebnis erzielen zu können, muss das Zielgewebe im Bereich der genannten Elektrodenabstände stark komprimiert vorliegen, was nur dadurch sichergestellt werden kann, wenn das Instrument vollständig über einen speziell hierfür vorgesehenen Verschlusshebel geschlossen ist. Es wird demzufolge zwischen drei Stellungen des für eine Darmanastomose geeigneten Versiegelungsinstruments unterschieden:
- Geöffnet und Instrumentenhälften (einschließlich Branchen) voneinander getrennt: In dieser Stellung können beide Instrumentenhälften unabhängig voneinander völlig frei im Raum bewegt werden. Insbesondere können die beiden Instrumentenhälften, auch wie beim Anlegen von Anastomosen erforderlich, unabhängig voneinander in Gewebevolumen, wie z.B. eröffnete Darmschnitte eingeführt werden.
- Geöffnet und Instrumentenhälften (einschließlich Branchen) verbunden bzw. scharnierartig gekoppelt: Die beiden Instrumentenhälften sind in dieser Stellung über eine Steck-/ Scharnierverbindung miteinander verbunden. Es kann somit eine Bewegung der beiden Instrumentenhälften um eine gemeinsame Schwenkachse erfolgen. Das Gewebe kann in dieser Stellung zwischen den (beabstandeten) Branchen noch verschoben werden. In dieser Offenstellung würde daher eine HF - Aktivierung zu einem unkontrollierten Fusionsergebnis führen, da die für die Fusionsprozesse erforderliche Flächenpressung an den Kontaktstellen zwischen den Elektroden und dem Gewebe nicht vorliegt. Dies stellt daher grundsätzlich ein Sicherheitsrisiko dar, weshalb ein Sicherheitskonzept erforderlich ist, das eine Aktivierung in dieser Instrumentenstellung ausschließt.
- Geschlossen (verspannt): Das Instrument wurde über den vorstehend genannten internen (Ver-) Schlusshebel geschlossen und das Zielgewebe mit einer vordefinierten (Minimal-) Klemmkraft zwischen den Branchen beaufschlagt und dabei ausreichend komprimiert. Das Zielgewebe kann in dieser Instrumentenstellung nicht mehr zwischen den Branchen verschoben werden. Die Instrumentenposition bezüglich des Gewebes kann nur nach erneutem Öffnen verändert werden. Nur in dieser Instrumentenstellung darf eine Aktivierung des HF-Stroms durch den Anwender möglich sein.

Beispielsweise ist aus der US 5,531,744 ein bipolares HF - Versiegelungsinstrument der vorliegenden Gattung bekannt. Dieses Instrument besteht aus zwei scharnierartig miteinander verbundenen Instrumentenhälften, an denen jeweils ein elektrischer Anschluss für ein elektrisches Verbinden der jeweiligen Instrumentenhälfte an einen HF-Generator angeordnet ist. Jede Instrumentenhälfte hat ferner einen Griff sowie eine Instrumentenbranche, die jeweils mit zumindest einer Elektrode bestückt sind. Schließlich ist an dem Griff einer Instrumentenhälfte ein in Grifflängsrichtung verschiebbarer Riegel gelagert, der mit einem Skalpell gekoppelt ist, das aus dem Griff längs der Instrumentenbranche herausgefahren werden kann, um ein zwischen den Instrumentenbranchen eingeklemmtes Gewebe zu zerschneiden.

Für das Einklemmen des Gewebes mit einer vorbestimmten Klemmkraft ist an der einen Instrumentenhälfte ein Schlusshebel mit einer daran angelenkten Klinke als Bestandteile des jeweiligen Griffs anscharniert, derart, dass beim manuellen Zusammenführen der beiden Instrumentenbranchen die Klinke in einen Bolzen an der gegenüberliegenden Instrumentenhälfte (gegenüberliegender Griff) einrasten kann, um dann durch Betätigen des Schusshebels eine Spannkraft auf den Bolzen und damit auf die (unter Zwischenlage des Gewebes) aneinander liegenden Instrumentenbranchen auszuüben.

Selbst wenn das HF - Versiegelungselement gemäß der US 5,531,744 in seine einzelnen Instrumentenhälften demontierbar sein sollte, bleiben diese beiden Instrumentenhälften ständig über jeweils eine eigene Stromleitung mit dem externen HF - Generator verbunden, wodurch die individuelle Bewegungsfreiheit auch in demontiertem Zustand des Instruments eingeschränkt ist. Des Weiteren sieht dieses bekannte Instrument keine Sicherungen gegen ein unbeabsichtigtes Beaufschlagen mit einem HF - Strom in einer nicht geschlossenen Stellung der Instrumentenbranchen vor.

Ferner offenbart die EP 2 436 330 ein bipolares chirurgisches HF-Instrument gemäß der vorliegenden Gattung, bei welchem eine Art Steuerschalter beispielsweise in Form eines Spannungs-Dehnungs-Messstreifens vorgesehen ist, der in einer elektrischen Steuer-/ Regelschaltung angeordnet ist, um ein Steuersignal zur Funktionsfreigabe eines weiteren, manuell zu betätigenden Leistungsschalters abzugeben, wenn eine vorbestimmte Schließkraft an den Gewebe-Klemmbranchen des Instruments erreicht wird. Dadurch lässt sich verhindern, dass mangelhaft eingeklemmtes Gewebe mit einem HF-Strom beaufschlagt wird, was zu einer fehlerhaften Gewebebehandlung führen könnte. Die Dokumente DE102008032511 und DE102006042985 offenbaren ebenfalls relevanten Stand der Technik.

### Kurzbeschreibung der Erfindung

Angesichts dessen ist es die Aufgabe der vorliegenden Erfindung, das bekannte HF - Versiegelungsinstrument der bipolaren Bauart so weiterzubilden, dass eine höhere Funktionalität und Betriebssicherheit erzielbar ist. Ein bevorzugtes Ziel ist es, die Möglichkeit der vollständig unabhängigen Manipulation beider Instrumentenhälften zu schaffen, ohne dass Fehlfunktionen bei der HF-Strombeaufschlagung auftreten. Diese Aufgabe sowie die vorteilhaften Ziele werden durch ein chirurgisches HF - Versiegelungsinstrument der bipolaren Bauart mit den Merkmalen des Anspruchs 1 gelöst/erreicht. Vorteilhafte Weiterbildungen der vorliegenden Erfindung sind Gegenstand der Unteransprüche.

Ein ggf. individuell zu beanspruchender Aspekt der Erfindung besteht darin, dass für den Fall, dass nur eine der beiden Instrumentenhälften über eine Stromleitung an eine Energiequelle, vorzugsweise einen externen HF-Generator oder dergleichen angeschlossen/anschließbar ist, der Leistungskontakt zwischen den beiden Instrumentenhälften für deren elektrisches Koppeln in montiertem Zustand (vorzugsweise als Federkontakt) für einen Kontaktschluss erst in oder nahe einer Schließstellung bzw. eines Schließstellungsbereichs für unterschiedliche Gewebedicken ausgebildet ist, wohingegen in der Offenstellung des (montierten) Instruments der Leistungskontakt nicht geschlossen ist. Auf diese Weise wird verhindert, dass in einer Offenstellung des Instruments ein Leistungsstrom (HF - Strom) zwischen den Elektroden beider Instrumentenbranchen durch das dazwischen eingeklemmte und komprimierte Gewebe fließt.

Der Leistungskontakt, der in die HF - Stromführleitung zwischengeschaltet ist, ist, wie bereits angedeutet, vorzugsweise ein Federkontakt, der folglich einen Kontaktschluss nicht nur bei einer einzigen bestimmten Schließstellung der zwei Instrumentenbranchen sondern über einen vorbestimmten Schließstellungsbereich entsprechend seines Federhubs bewirkt. Daher kann das Instrument für unterschiedliche Gewebedicken verwendet werden, ohne darauf individuell eingestellt werden zu müssen.

Gemäß der Erfindung ist das bipolare HF - Instrument mit einem instrumenteninternen Steuerschalter (nicht in die HF - Leistungsstromführleitung zwischengeschaltet) ausgerüstet, der dafür angepasst ist, um gleich durch/mit Zusammenbau der beiden Instrumentenhälften aktiviert zu werden, wodurch eine elektrische Strombeaufschlagungsmöglichkeit von einer Energiequelle, vorzugsweise einem externen HF-Generator an die beiden Instrumentenbranchen bzw. den Leistungskontakt freigegeben wird. Vorzugsweise ist der Steuerschalter in die Steuerleitung zwischen einem Hand- oder Fußschalter und einer externen Energiequelle zwischengeschaltet oder er gibt parallel hierzu ein elektrisches Signal an eine (ggf. externe) Steuerschaltung ab, welche die Erzeugung/Bereitstellung eines elektrischen HF - Stroms steuert/regelt. D.h. der erfindungsgemäße Steuerschalter ist bevorzugt nicht in den Leistungsstrom zu den Elektroden zwischengeschaltet sondern hat einen Sperr- oder Freigabeeinfluss vorzugsweise auf die elektrische Steuerung der Energiequelle. Selbst für den Fall, dass zumindest die eine, grundsätzlich an die Energiequelle angeschlossene Instrumentenbranche bzw. die darin eingelegten Elektroden gewollt oder ungewollt einen elektrischen Kontakt zu der Energiequelle auch in Offenstellung erhalten, würde daher die manuelle Betätigung eines Hand- oder Fußschalters zur Beaufschlagung der Elektroden mit HF - Strom solange nicht funktionieren, bis der (ggf. weitere) Steuerschalter durch den Montagevorgang geschlossen wird.

Jede der beiden vorstehend genannten Maßnahmen, nämlich die Anordnung des Leistungskontakts und/oder des Steuerschalters sollen eine unkontrollierte Thermofusion im Fall einer Offenstellung des Instruments oder im Fall von frei (unabhängig) bewegbaren manipulierbaren Instrumentenhälften (unmontierter Zustand) verhindern. Da die beiden genannten Maßnahmen auf unterschiedliche Mechanismen des Instruments einwirken, d.h. der Leistungskontakt ist in die Leistungsstromleitung zwischengeschaltet, wohingegen der Steuerschalter die Verbindung zwischen Hand- oder Fußschalter und Steuerschaltung schließt/unterbricht oder alternativ hierzu ein (paralleles) Steuersignal an die Energiequellensteuerung ausgibt, können diese unabhängig voneinander oder gemeinsam in einem HF - Versiegelungsinstrument dieser Gattung verbaut werden, wobei im letzteren Fall ein zusätzlicher "fail - safe" - Effekt erzielbar ist.

Gemäß einem weiteren, ggf. unabhängigen Aspekt der vorliegenden Erfindung ist der Steuerschalter in eine elektrische Steuer- oder Signalleitung zwischen einem Hand- oder Fußschalter und der Steuerung vorzugsweise der Energiequelle zwischengeschaltet und befindet sich damit in einer logischen UND Schaltung mit dem Hand- oder Fußschalter, um beispielsweise in Schließstellung des Instruments oder bei erfolgter Montage die Steuerleitung für eine Stromfreigabe zur (danach erfolgenden) wahlweisen HF - Strombeaufschlagung der Elektroden mittels des Hand- oder Fußschalters zu schließen. D.h., dass eine manuelle Betätigung des Hand- oder Fußschalters zur Strombeaufschlagung der in den Instrumentenbranchen eingelagerten Elektroden solange erfolglos ist, bis die Steuerleitung durch den Steuerschalter geschlossen oder alternativ hierzu ein entsprechendes (paralleles) Freigabesignal vom Steuerschalter an der Steuerung anliegt.

Die vorliegende Erfindung wird nachfolgend anhand eines bevorzugten Ausführungsbeispiels unter Bezugnahme auf die begleitenden Figuren näher erläutert.
Fig. 1 zeigt die Seitenansicht eines HF - Versiegelungsinstruments gemäß einem bevorzugten Ausführungsbeispiel der vorliegenden Erfindung,
Fig. 2 zeigt eine erste Perspektivenansicht des HF - Versiegelungsinstruments ohne Griffschalen sowie schräg aus seitlicher Richtung,
Fig. 3 zeigt eine zweite Perspektivenansicht des HF - Versiegelungsinstruments ohne Griffschalen sowie schräg aus distaler Richtung,
Fig. 4 zeigt eine dritte Perspektivenansicht des HF - Versiegelungsinstruments ohne Griffschalen sowie schräg aus proximaler Richtung,
Fig. 5 zeigt die Perspektivenansicht des proximalen Endabschnitts des HF - Versiegelungsinstruments in Vergrößerung und
Fig. 6 zeigt Rückansicht des proximalen Endabschnitts des HF - Versiegelungsinstruments in Vergrößerung.

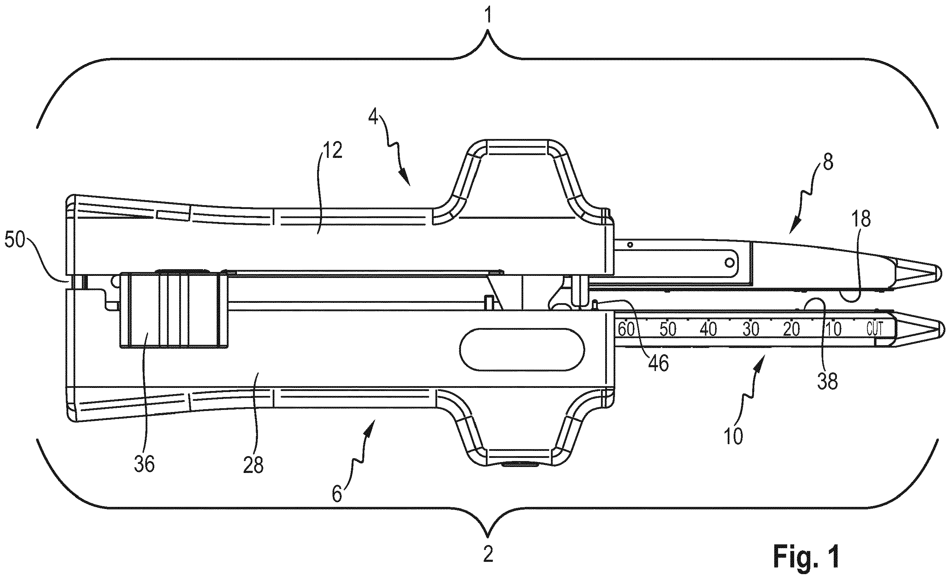

Das in der Fig. 1 in Seitenansicht dargestellte HF - Versiegelungsinstrument der bipolaren Bauart gemäß einem bevorzugten Ausführungsbeispiel der vorliegenden Erfindung hat grundsätzlich zwei trennbar miteinander scharnierartig verbundene (verbindbare) Instrumentenhälften 1, 2, die jeweils aus einer Griffeinheit 4, 6 und einer in Instrumentenlängsrichtung distal hierzu angeordneten Instrumentenbranche 8, 10 bestehen.

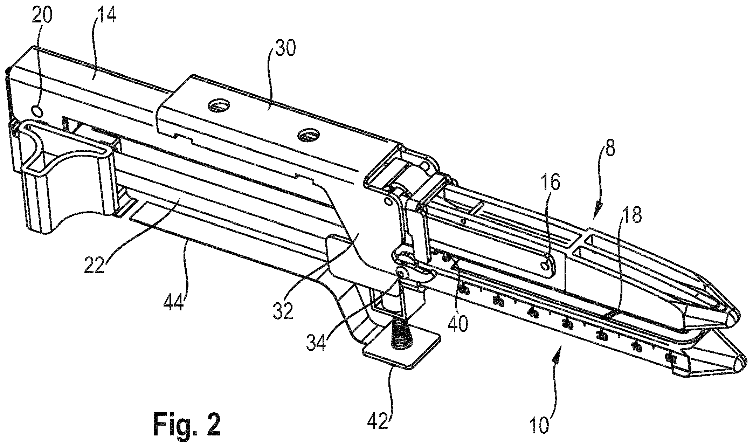

Die Griffeinheit 4 der einen, gemäß Fig. 1 oberen Instrumentenhälfte 1 hat eine ergonomisch geformte Griffschale 12 beispielsweise aus einem Kunststoff- oder Blechmaterial, die gemäß Fig. 2 über einen axialen Verlängerungsschaft 14 der Instrumentenbranche 8 dieser einen Instrumentenhälfte 1 schützend übergesteckt ist. Unterhalb der Griffschale 12 ist an dem Verlängerungsschaft 14 der einen (oberen) Instrumentenbranche 8 gemäß der Fig. 2 ein Schließhebel 30 angeordnet, der an seinem distalen Ende schwenkbar relativ zur Griffschale 12 am Verlängerungsschaft 14 gelagert ist und ferner eine (einstückig mit dem Schließhebel ausgebildete) Eingriffsklinke 32 hat, die seitlich vom Verlängerungsschaft 14 in Richtung hin zur anderen (gemäß Fig. 2 unteren) Instrumentenhälfte 2 vorragt. Der Schließhebel 30 samt Klinke 32 stellt dabei einen Teil eines Instrumenten - Schließmechanismus dar, wie es nachfolgend noch beschrieben wird.

Der Verlängerungsschaft 14 der einen (gemäß Fig. 2 oberen) Instrumentenbranche 8 hat an seinem distalen Ende eine quer verlaufende Durchgangsbohrung 16, in die ein Schwenkbolzen (nur angedeutet) eingesteckt ist, an dem die (obere) Instrumentenbranche 8 wippenartig, d.h. mittig angelenkt ist. An einer der gegenüberliegenden anderen (unteren) Instrumentenbranche 10 zugewandten (unteren) Längsseite der einen (oberen) Instrumentenbranche 8 ist zumindest eine, vorzugsweise mehrere Elektroden18 eingelagert (in der Fig. 2 nur andeutungsweise erkennbar), die über eine nicht weiter dargestellte elektrische Leistungsstromleitung mit einer Kontaktplatte (nicht im Einzelnen dargestellt) verbunden sind, die am proximalen Endabschnitt der einen (oberen) Instrumentenbranche 8 angeordnet ist und in Richtung hin zur (unteren) anderen Instrumentenbranche 10 freiliegt.

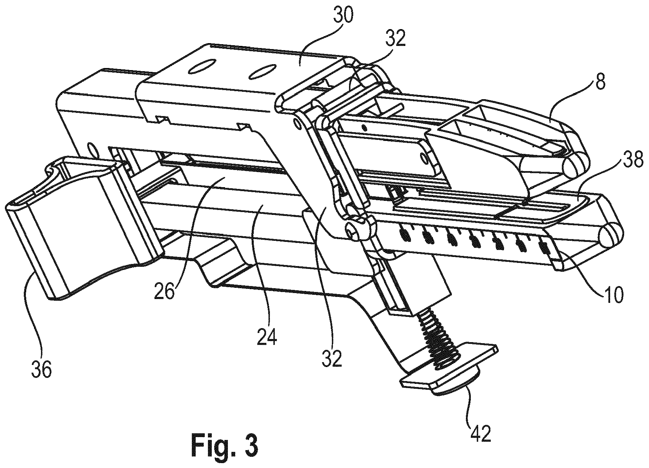

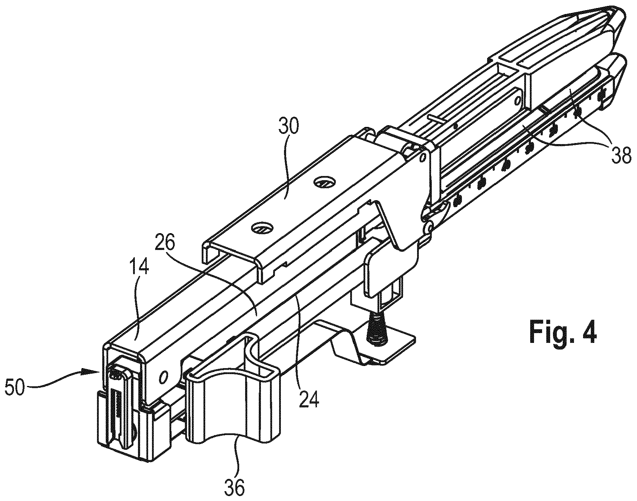

Wie ebenfalls aus der Fig. 2 zu entnehmen ist, hat die eine (obere) Instrumentenhälfte 1 im proximalen Bereich von deren Griffeinheit 4 bzw. Verlängerungsschaft 14 eine weitere Quer - Durchgangsbohrung mit eingestecktem Scharnierbolzen 20, an dem die andere (untere) Instrumentenhälfte 2 anscharniert/anscharnierbar ist. Auch diese andere (untere) Instrumentenhälfte 2 hat die distale Instrumentenbranche 10 mit einem nach proximal sich erstreckenden Verlängerungsschaft 22, der gemäß der Fig. 3 oder 4 eine längsschlitzförmige Gleitaufnahme 24 für ein axial verschiebbares Schneid-/Trennmesser 26 bildet. Am (gemäß Fig. 2 unteren) Verlängerungsschaft 22 ist auch die (untere) Griffeinheit 6 vorgesehen bestehend aus einer, den (gemäß Fig. 1 unteren) Verlängerungsschaft 22 umgebenden Griffschale 28 sowie einem Rastelement 34, mit welchen die Eingriffsklinke 32 des (oberen) Schließhebels 30 der einen (oberen) Instrumentenhälfte 1 in Spanneingriff bringbar ist, um die beiden Instrumentenhälften 1, 2 und insbesondere die beiden Instrumentenbranchen 8, 10 gegeneinander mit einer bestimmten Klemmkraft vorzuspannen.

Das Trennmesser 26 ist ferner mittels eines seitlich von der (unteren) Griffschale 28 vorragenden Riegels 36 axial aus einer in Fig. 1 gezeigten Parkposition innerhalb der Griffschale 28 in eine Schneidposition im Bereich der Instrumentenbranche 10 verschiebbar. Das Trennmesser 26 kann hierfür als rein mechanisch wirkendes Skalpell oder als eine elektrisch schneidende Trennplatte ausgeformt sein, die mit Verschieben aus ihrer Parkposition ein zwischen den Instrumentenbranchen eingeklemmtes Gewebe durchtrennt.

Die andere (untere) Instrumentenbranche 10 ist an ihrer der einen (oberen) Instrumentenbranche 8 zugewandten Längsseite ebenfalls mit zumindest einer Elektrode oder mehreren Elektrodensegmenten 38 bestückt, welche mit einem (nicht weiter dargestellten) elektrischen Anschluss über eine Leistungsstromleitung elektrisch verbunden sind. An diesem elektrischen Anschluss, der vorzugsweise am proximalen Ende der anderen (unteren) Instrumentenhälfte 2 angeordnet ist, ist eine ebenfalls nicht weiter gezeigte Energiequelle, beispielsweise ein HF - Generator angeschlossen oder anschließbar, um die Elektrode 38 der anderen (unteren) Instrumentenhälfte 2 durch Betätigen eines Handschalters 42 mit einem HF - Strom zu beaufschlagen. Im vorliegenden Ausführungsbeispiel ist der Handschalter 42 in der Griffeinheit 6 der anderen (unteren) Instrumentenhälfte 2 integriert (siehe hierzu insbesondere Fig. 1). Es kann alternativ aber auch ein externer Fußschalter vorgesehen sein, wobei in diesem Fall der in den Figuren dargestellte Handschalter 42 beispielsweise in den elektrischen Anschluss geändert sein kann. In beiden Fällen ist der Hand- oder Fußschalter 42 über eine Steuerleitung 44 mit der elektronischen Steuerung der Energiequelle verbunden, um diese entsprechend zu aktivieren.

Des Weiteren ist in der anderen (unteren) Instrumentenhälfte 2 ausgehend von dem elektrischen Anschluss eine zweite elektrische Leistungsstromleitung verlegt (nicht gezeigt), die in einem am proximalen Ende der (unteren) Instrumentenbranche 10 an deren oberen Längsseite angeordneten Leistungskontakt 40 endet.

Dieser Leistungskontakt 40 besteht vorliegend aus einem am proximalen Endbereich der (unteren) Instrumentenbranche 10 federnd gelagerten Kontaktstift 46 (kann auch eine Federzunge oder dergleichen Federkontakt sein), der bei Schließen des Instruments, d.h. beim aufeinander zu Bewegen der zwei Instrumentenbranchen 8, 10 ab einer bestimmten Relativ - Schwenkposition mit der (oberen) freiliegenden Kontaktfläche in elektrischen Kontakt kommt und damit die (oberen) Elektrode(n) 18 mit dem elektrischen Anschluss an der anderen (unteren) Instrumentenhälfte 2 verbindet.

Der Kontaktstift 46 ist hierfür so gelagert, dass er einen bestimmten Hub ausführen kann, sodass er an der (oberen) Kontaktfläche bereits elektrisch leitend anliegt, bevor das Instrument seine maximal mögliche Schließ - Schwenkposition erreicht hat. D.h. der elektrische Leistungskontakt 40 ist über einen bestimmten Schwenk - Schließbereich (nachfolgend als Schließstellungsbereich bezeichnet) der beiden Instrumentenbranchen 8, 10 geschlossen, wobei nur in Offenposition des Instruments der Leistungskontakt 40 garantiert nicht geschlossen ist. Vorzugsweise hat der Kontaktstift 46 einen Federhub, der einen Schließstellungsbereich der beiden Instrumentenbranchen 8, 10 (Branchenabstand in Schließstellung) zwischen 0.1mm und 1.5mm Spaltweite zwischen den Branchen 8, 10 ermöglicht. Alternativ hierzu ist es aber auch möglich, den Leistungskontakt 40 so zu gestalten, dass dieser in (oder unmittelbar vor) nur einer einzigen Schließstellung der beiden Instrumentenbranchen 8, 10 elektrisch geschlossen ist.

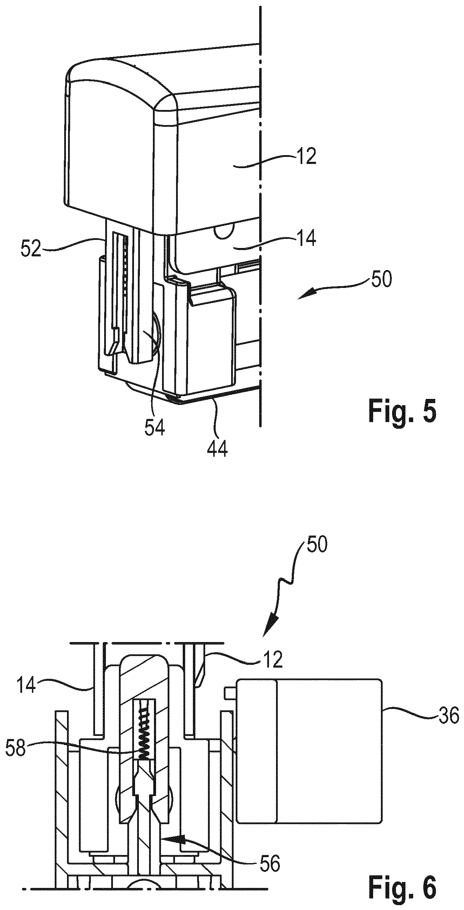

Zusätzlich oder alternativ zu dem vorstehend beschrieben, die (obere) Elektrode(n) 18 mit dem Anschluss der einen (oberen) Instrumentenhälfte verbindenden Leistungskontakt 40 ist an der einen (oberen) Instrumentenhälfte 1 vorzugsweise am proximalen Ende der Griffeinheit 4 ein weiterer Steuerschalter 50 angeordnet, wie er insbesondere in den Fig. 4 bis 6 dargestellt ist. Dieser Steuerschalter 50 (Montageüberwachungsschalter) kann in einem nicht beanspruchten Beispiel dazu dienen, das Instrument in der geöffneten Stellung (gemäß sämtlicher Figuren) und in der geschlossenen Stellung zu detektieren. Hierbei dient die (dargestellte) offene Stellung der Manipulation und dem Gewebe Erfassen mit dem Instrument. In dieser Stellung liegt das Gewebe folglich nicht oder unzureichend komprimiert zwischen den beiden Instrumentenbranchen 8, 10. Eine Aktivierung des HF - Stroms in dieser Stellung könnte daher zu einem unerwünschten (unvollständigen) Fusionsergebnis führen und stellt daher ein enormes Sicherheitsrisiko für den Patienten dar. Eine vollständige Versiegelung (Fusion) kann nur unter einer bestimmten Kompression der zu verbindenden Gewebeteile erfolgen. Diese wird im erfindungsgemäßen Instrument durch Aufbringen der vorstehend genannten Mindestklemmkraft beim Schließen bzw. mit Erreichen der Schließstellung erzielt. Es muss daher sichergestellt sein, dass diese Schließstellung auch tatsächlich erreicht ist, bevor eine HF - Stormbeaufschlagung der Elektroden 18, 38 erfolgt. Genau hierfür könnte der Steuerschalter 50 vorgesehen werden, der durch/mit Betätigen des Schließhebels 30 des eingesetzten Schließmechanismus über ein Tastelement 52 mit Erreichen des seiner Schließstellung aktiviert wird.

In diesem Fall läge der technische Sachverhalt vor, wonach der Steuerschalter 50 zum gleichen Zweck dient, wie der zuvor beschriebene Leistungsschalter 40, jedoch an einer anderen Funktionsstelle des Instruments (nämlich in der Steuerung) angeordnet ist, wohingegen der Leistungsschalter 40 in die Leistungsstromleitung zwischengeschaltet ist. Erfindungsgemäß ist es aber vorgesehen, den Steuerschalter 50 bzw. dessen Tastelement 52 so zu platzieren, dass er bereits mit dem scharnierförmigen Zusammenbau der beiden Instrumentenhälften 1, 2 betätigt wird. In diesem Fall richtet sich seine Funktion darauf, eine Bestromung zumindest der Elektrode(n) 38 der den elektrischen Anschluss aufweisenden Instrumentenhälfte 2 solange zu verhindern (könnte in diesem Fall ungewollt als monopolare Elektrode fungieren), bis das Instrument in fertig montiertem Funktionszustand gebracht ist.

Im Konkreten ist der erfindungsgemäße Steuerschalter 50 vorzugsweise ein Folienschalter, der am proximalen Ende der einen (oberen) Instrumentenhälfte 1 an deren Verlängerungsschaft 14 montiert ist, derart, dass sich dessen Betätigungsfeld 54 in Axialrichtung des Verlängerungsschafts 14 ausrichtet. In einem Proximalabstand zu dem Betätigungsfeld 54 des Folienschalters 50 ist das Tastelement 52 in Form eines an der einen (oberen) Instrumentenhälfte 1 quer verschiebbar gelagerten Linearschiebers angeordnet, der auf einer stegförmigen Linearführung 56 an der einen (unteren) Instrumentenhälfte 2 gleitend sitzt. Im Konkreten ist am proximalen Ende des oberen Verlängerungsschafts 12 ein Quersteg oder Querleiste 56 angeformt, der sich in Richtung hin zur anderen (unteren) Instrumentenhälfte 2 erstreckt und auf dem das riegelförmige Tastelement 52 verschiebbar sitzt. Das Tastelement 52 ist ferner mittels einer Feder 58 in eine Richtung vorgespannt, in welcher der Steuerschalter 50 unbetätigt ist. An der dem Bedienfeld 54 zugewandten Seite des Tastelements 52 ist ein nicht weiter dargestellter Vorsprung ausgeformt, der mit Verschieben des Tastelements 52 gegen die Feder 58 auf das Bedienfeld 54 drückt und so den Steuerschalter 50 betätigt.

Hierdurch wird die Steuerleitung 44 zwischen dem Handschalter 42 und der Steuerung der Energiequelle, welche über den Steuerschalter 50 führt geschlossen, sodass ein an den Handschalter 42 manuell erzeugtes Betätigungssignal zur Elektrodenbestromung über den Steuerschalter 50 an die Energiequelle geführt werden kann. Alternativ ist es aber auch möglich, dass ein elektrisches Signal vom Steuerschalter 50 bei dessen Betätigung durch das Tastelement 52 ausgegeben wird, das an eine nicht weiter gezeigte Steuerschaltung der externen Energiequelle angelegt wird, die daraufhin den HF - Strom grundsätzlich frei schaltet. Erst jetzt kann der HF - Strom durch Betätigen des Hand- oder Fußschalters wahlweise abgerufen und die Elektroden für eine Thermofusion bestromt werden. In diesem Fall wäre der Steuerschalter 50 parallel zum Hand- oder Fußschalter mit der Energiequellensteuerung verbunden.

Die Vorteile der vorliegenden Erfindung insbesondere bei Einsatz des Leistungs- und Steuerschalters lassen sich demnach wie Folgt zusammenfassen:
- Das Instrument kann in der getrennten (unmontierten) wie auch in der verbundenen (montierten) aber noch geöffneten Stellung der beiden Instrumentenhälften 1, 2 nicht elektrisch aktiviert werden. Dadurch wird eine versehentliche Aktivierung zur Unzeit oder eine undefinierte Versiegelung bei nicht ausreichend komprimiertem Gewebe vermieden.
- Zur Auslösung muss das Instrument montiert sowie geschlossen und der HF - Strom durch den Anwender aktiv über den Hand- oder Fußschalter 42 abgerufen werden. Dies bedeutet, dass sich der Steuerschalter 50 bei der gezeigten Ausführungsform in einer logischen UND Schaltung mit dem Hand- oder Fußschalter 30 für die HF - Aktivierung befindet, sodass nur dann, wenn beide Schalter 30, 50 betätigt sind, eine HF - Aktivierung erfolgt.
- Der Leistungsschalter 40 und der Steuerschalter 50 wirken an unterschiedlichen Funktionspunkten des Instruments. Insbesondere ist der Leistungsschalter in den Leistungsstromkreis zwischengeschaltet, wohingegen der Steuerschalter 50 an die Steuerung der Energiequelle anschließbar ist. Beide Schalter bewirken demnach auf unterschiedliche Weise ein Blockieren der Bestromung der Elektroden 18, 38 für den Fall, dass sich das Instrument nicht in Schließstellung befindet (Leistungsschalter 40) bzw. nicht montiert ist (Steuerschalter 50).. Daher können beide Schalter unabhängig voneinander wahlweise eingebaut sein, wobei jedoch beim gleichzeitigen Einbau beider Schalter zusätzlich eine "fail-safe" Funktion erreicht wird.

Abschließend sei noch darauf hingewiesen, dass der Steuerschalter 50 mit einem weiteren Betätigungsfeld ausgebildet sein kann (oder es ist ein weiterer Steuerschalter vorgesehen), der vom Trennmesser 26 oder dessen Betätigungsriegel 36 aktivierbar ist. In diesem Fall signalisiert dieser weitere Steuerschalter die Parkposition des Trennmessers als weitere Voraussetzung dafür, dass HF - Strom für die Elektroden abrufbar ist.

## Patentansprüche

1. Bipolares HF-Instrument, mit zwei Instrumentenhälften (1, 2), jeweils bestehend aus einer Elektroden-bestückten, langgestreckten Instrumentenbranche (8, 10), die relativ zueinander beweglich gelagert oder lagerbar sind, um in eine Öffnungs- und eine Schließstellung betätigt zu werden, sowie aus einer Griffeinheit (4, 6), von denen jeweils eine einer Instrumentenbranche (8, 10) zugeordnet ist, um die Instrumentenbranchen (8, 10) manuell zu betätigen sowie auf die Instrumentenbranchen (8, 10) in Schließstellung eine vorbestimmte Schließkraft aufzubringen, **gekennzeichnet durch** einen instrumenteninternen Steuerschalter (50), der dafür angepasst ist, um als Montagesicherung durch Zusammenbau der beiden Instrumentenhälften (1, 2), so dass das Instrument in einen fertig montierten Funktionszustand gebracht ist, zwangsläufig automatisch aktiviert zu werden, um einen elektrischen Strom von einer Energiequelle, vorzugsweise einem externen HF-Generator, an die beiden Instrumentenbranchen (8, 10) freizugeben, wobei der Steuerschalter (50) an der Griffeinheit (4) der einen Instrumentenhälfte (1) an einer proximalen Instrumentenlängsposition platziert ist.

2. Bipolares HF-Instrument nach Anspruch 1, **dadurch gekennzeichnet, dass** an nur einer der beiden Instrumentenbranchen (8, 10) ein Anschluss für eine externe Energiequelle, vorzugsweise einen HF-Generator, aktiv ist und die andere Instrumentenbranche (8) über einen instrumenteninternen Leistungskontakt (40) mit der einen, den aktiven Anschluss aufweisenden Instrumentenbranche (10) elektrisch gekoppelt oder koppelbar ist, und dass der Leistungskontakt (40) für einen Kontaktschluss während einer Schließbewegung erst mit oder kurz vor Erreichen der Schließstellung oder eines für unterschiedliche Gewebedicken vorbestimmten Schließstellungsbereichs ausgebildet ist, der jedoch in Offenstellung des Instruments nicht geschlossen ist.

3. Bipolares HF-Instrument nach Anspruch 2, **dadurch gekennzeichnet, dass** der Leistungskontakt (40) ein Federkontakt ist.

4. Bipolares HF-Instrument nach Anspruch 3, **dadurch gekennzeichnet, dass** der Federkontakt einen federvorgespannten Kontaktstift (46) oder eine Federzunge hat, der/die sich von einer der Instrumentenhälften (2) in Richtung hin zur anderen Instrumentenhälfte (1) erstreckt, um von dieser betätigt werden zu können.

5. Bipolares HF-Instrument nach einem der Ansprüche 2 bis 4, **dadurch gekennzeichnet, dass** der Leistungskontakt (40) im Bereich der Instrumentenbranchen (8, 10) außerhalb deren Gewebeklemmabschnitte angeordnet ist.

6. Bipolares HF-Instrument nach einem der Ansprüche 1 bis 5, **dadurch gekennzeichnet, dass** der Steuerschalter (50) ein Tastschalter, vorzugsweise ein Folienschalter ist, der an einer der Instrumentenhälften (1) angeordnet und von einem Tastelement (52) an der einen Instrumentenhälfte (1) betätigbar ist.

7. Bipolares HF-Instrument nach einem der Ansprüche 1 bis 6, **dadurch gekennzeichnet, dass** der Steuerschalter (50) über eine elektrische Leitung mit einer Steuerung der Energiequelle verbunden oder verbindbar ist, um an diese ein Montage-erfolgt-Signal für eine Stromfreigabe zur wahlweisen HF - Strombeaufschlagung der Elektroden mittels eines weiteren Hand- oder Fußschalters abzugeben.

8. Bipolares HF-Instrument nach einem der Ansprüche 1 bis 6, **dadurch gekennzeichnet, dass** der Steuerschalter (50) in eine Steuerleitung zwischen einem Hand- oder Fußschalter (42) und einer Steuerung der Energiequelle zwischengeschaltet ist.

9. Bipolares HF-Instrument nach Anspruch 3 oder 4, **dadurch gekennzeichnet, dass** der Federkontakt (40) so ausgebildet und/oder am Instrument angeordnet ist, dass er einen Kontaktschluss federelastisch über einen vorbestimmten Schließstellungs/-bewegungsbereich der Instrumentenbranchen (8, 10) entsprechend seines Federhubs bewirkt.

10. Bipolares HF-Instrument nach Anspruch 9, **dadurch gekennzeichnet, dass** die beiden miteinander in elektrischen Kontakt bringbaren Kontaktelemente (46) nach Kontaktschluss über den vorbestimmten Schließstellungs/- bewegungsbereich relativ zueinander unbeweglich gehalten sind.

11. Bipolares HF-Instrument nach Anspruch 9 oder 10, **dadurch gekennzeichnet, dass** der Federkontakt (40) so ausgebildet und/oder am Instrument angeordnet ist, dass der Kontaktschluss in einem Mittenabschnitt des Schließstellungs/- bewegungsbereichs der Instrumentenbranchen (8, 10) hergestellt und dann bis Erreichen der maximalen Schließstellung beibehalten wird.

## Claims

1. A bipolar RF instrument comprising two instrument halves (1, 2), each consisting of an electrode-equipped, elongate instrument branch (8, 10), wherein the instrument branches (8, 10) are supported or are adapted to be supported such that they are movable relative to one another so as to be operated for moving to an open and a closed position, and of a handle unit (4, 6), each instrument branch (8, 10) having associated therewith a respective handle unit (4, 6) for manual operation of the instrument branches (8, 10) and for applying to the instrument branches (8, 10) a predetermined closing force at the closed position, **characterized by** an instrument-internal control switch (50), which is adapted to be inevitably automatically activated as a mounting monitoring means due to the assembly of the two instrument halves (1, 2), so that the instrument is brought into a final mounted operating state, so as to enable the supply of electric current from an energy source, preferably an external RF generator, to the two instrument branches (8, 10), wherein the control switch (50) is positioned on the handle unit (4) of said one instrument half (1), at a proximal instrument length position.

2. A bipolar RF instrument according to claim 1, **characterized in that** a terminal for an external energy source, preferably an RF generator, is active on only one of the two instrument branches (8, 10) and the other instrument branch (8) is electrically coupled or adapted to be electrically coupled via an instrument-internal power contact (40) with the instrument branch (10) including the active terminal, and **in that** the power contact (40) is configured for contact closure during a closing movement only when the closure position or a closure position region predetermined for different tissue thicknesses is reached, or shortly before that, but is not closed at the open position of the instrument.

3. A bipolar RF instrument according to claim 2, **characterized in that** the power contact (40) is a spring contact.

4. A bipolar RF instrument according to claim 3, **characterized in that** the spring contact is a spring-loaded contact pin (46) or a flexible tongue extending from one of the instrument halves (2) in the direction of the other instrument half (1) so that it can be operated by the latter.

5. A bipolar RF instrument according to one of the claims 2 to 4, **characterized in that** the power contact (40) is arranged in the area of the instrument branches (8, 10) outside the tissue clamping portions thereof.

6. A bipolar RF instrument according to one of the claims 1 to 5, **characterized in that** the control switch (50) is a key switch, preferably a membrane switch, arranged on one of the instrument halves (1) and operable by a key element (52) on said one instrument half (1).

7. A bipolar RF instrument according to one of the claims 1 to 6, **characterized in that** the control switch (50) is connected to or adapted to be connected to a control unit of the energy source via an electric line so as to transmit, by means of an additional manual switch or foot switch, an "assembly completed" signal to the energy source control unit for enabling a supply of current for selectively applying an RF current to the electrodes.

8. A bipolar RF instrument according to one of the claims 1 to 6, **characterized in that** the control switch (50) is interposed in a control line between a manual switch or a foot switch (42) and a control unit of the energy source.

9. A bipolar RF instrument according to claim 3 or 4, **characterized in that** the spring contact (40) is configured and/or arranged on the instrument such that it causes contact closure resiliently over a predetermined closure position/motion range of the instrument branches (8, 10) in accordance with its spring stroke.

10. A bipolar RF instrument according to claim 9, **characterized in that,** after contact closure over a predetermined closure position/motion range, the two contact elements (46), which are adapted to be moved into electric contact with one another, are held such that they are not movable relative to one another.

11. A bipolar RF instrument according to claim 9 or 10, **characterized in that** the spring contact (40) is configured and/or arranged on the instrument such that contact closure is established in a middle region of the closure position/motion range of the instrument branches (8, 10) and is then maintained until the maximum closure position has been reached.

## Revendications

1. Instrument HF bipolaire, avec deux moitiés d'instrument (1, 2), constituées respectivement d'une branche d'instrument (8, 10) de forme allongée équipée d'électrodes, qui sont montées ou peuvent être montées de manière mobile l'une par rapport à l'autre, afin d'être actionnées dans une position d'ouverture et une position de fermeture, ainsi que d'une unité de préhension (4, 6), dont respectivement l'une est associée à une branche d'instrument (8, 10), afin d'actionner manuellement les branches d'instrument (8, 10) ainsi que d'appliquer une force de fermeture prédéfinie sur les branches d'instrument (8, 10) dans la position de fermeture, **caractérisé par** un interrupteur de commande (50) à l'intérieur de l'instrument, qui est adapté, en tant que sécurité de montage, par assemblage des deux moitiés d'instrument (1, 2), de sorte que l'instrument est amené dans un état de fonctionnement entièrement monté, à être activé automatiquement de manière obligatoire, afin de libérer un courant électrique provenant d'une source d'énergie, de préférence d'un générateur HF externe, sur les deux branches d'instrument (8, 10), dans lequel l'interrupteur de commande (50) est placé sur l'unité de préhension (4) d'une des moitiés d'instrument (1) sur une position longitudinale proximale d'instrument.

2. Instrument HF bipolaire selon la revendication 1, **caractérisé en ce qu'**un raccord pour une source d'énergie externe, de préférence un générateur HF, est actif sur une seule des deux branches d'instrument (8, 10) et l'autre branche d'instrument (8) est couplée ou peut être couplée électriquement à l'une des branches d'instrument (10) présentant le raccord actif par l'intermédiaire d'un contact de puissance (40) à l'intérieur de l'instrument, et que le contact de puissance (40) est réalisé pour une liaison de contact pendant un déplacement de fermeture uniquement avec ou peu avant l'atteinte de la position de fermeture ou d'une zone de position de fermeture prédéfinie pour différentes épaisseurs de tissu, qui n'est cependant pas fermée dans la position d'ouverture de l'instrument.

3. Instrument HF bipolaire selon la revendication 2, **caractérisé en ce que** le contact de puissance (40) est un contact élastique.

4. Instrument HF bipolaire selon la revendication 3, **caractérisé en ce que** le contact élastique présente une broche de contact (46) précontrainte par un ressort ou une languette élastique, qui s'étend d'une des moitiés d'instrument (2) en direction de l'autre moitié d'instrument (1), afin de pouvoir être actionnée par celle-ci.

5. Instrument HF bipolaire selon l'une quelconque des revendications 2 à 4, **caractérisé en ce que** le contact de puissance (40) est disposé dans la zone des branches d'instrument (8, 10) à l'extérieur des parties de serrage de tissu de celles-ci.

6. Instrument HF bipolaire selon l'une quelconque des revendications 1 à 5, **caractérisé en ce que** l'interrupteur de commande (50) est un interrupteur à poussoir, de préférence un interrupteur à feuilles, qui est disposé sur une des moitiés d'instrument (1) et peut être actionné par un élément à poussoir (52) sur une des moitiés d'instrument (1).

7. Instrument HF bipolaire selon l'une quelconque des revendications 1 à 6, **caractérisé en ce que** l'interrupteur de commande (50) est relié ou peut être relié à une commande de la source d'énergie par l'intermédiaire d'une ligne électrique, afin de délivrer à celle-ci un signal de montage effectué pour une libération de courant pour l'alimentation en courant HF sélective des électrodes au moyen d'un autre interrupteur manuel ou au pied.

8. Instrument HF bipolaire selon l'une quelconque des revendications 1 à 6, **caractérisé en ce que** l'interrupteur de commande (50) est intercalé dans une ligne de commande entre un interrupteur manuel ou au pied (42) et une commande de la source d'énergie.

9. Instrument HF bipolaire selon la revendication 3 ou 4, **caractérisé en ce que** le contact élastique (40) est réalisé et/ou disposé sur l'instrument de sorte qu'il provoque une liaison de contact de manière élastique sur une zone de déplacement/position de fermeture prédéfinie des branches d'instrument (8, 10) en fonction de sa course de ressort.

10. Instrument HF bipolaire selon la revendication 9, **caractérisé en ce que** les deux éléments de contact (46) pouvant être amenés en contact électrique l'un avec l'autre sont retenus de manière immobile l'un par rapport à l'autre sur la zone de déplacement/position de fermeture prédéfinie après la liaison de contact.

11. Instrument HF bipolaire selon la revendication 9 ou 10, **caractérisé en ce que** le contact élastique (40) est réalisé et/ou disposé sur l'instrument de sorte que la liaison de contact est établie dans une partie centrale de la zone de déplacement/position de fermeture des branches d'instrument (8, 10) et est ainsi conservée jusqu'à l'atteinte de la position de fermeture maximale.
